# EUROPÄISCHE PATENTANMELDUNG

(11) **EP 4 509 054 A1**
(43) Veröffentlichungstag der Anmeldung: **19.02.2025**
(21) Anmeldenummer: 23191290.8
(22) Anmeldetag: 14.08.2023
(51) Int. Cl.: A61B 6/00

(54) **VERFAHREN UND VORRICHTUNG ZUR NACHBEARBEITUNG VON RADIOGRAMMEN**

(71) Anmelder: Siemens Healthineers AG, 91301 Forchheim (DE)
(72) Erfinder: Beister, Marcel, 91058 Erlangen (DE); Herbst, Magdalena, 91056 Erlangen (DE); Ritschl, Ludwig, 96155 Buttenheim (DE); Schäfer, Jamin, 91052 Erlangen (DE)
(74) Vertreter: Siemens Healthineers Patent Attorneys

(57) **Zusammenfassung**

Die Erfindung betrifft ein Verfahren zur Nachbearbeitung von Radiogrammen (R) aus Radiographiedaten, welche spektrale Aufnahmen eines Motivs (M) eines Objekts (P) umfassen, das Verfahren umfassend die Schritte:
- Erstellen eines ersten Bildes (B1) aus den Radiogrammen (R), in dem ein erstes Material des Objekts (P), welches andere spektrale Schwächungseigenschaften besitzt als ein zweites Material des Objekts (P), hervorgehoben wird und das zweite Material unterdrückt wird,
- Erstellen eines zweiten Bildes (B2) aus den Radiogrammen (R), in dem das zweite Material hervorgehoben wird und das erste Material unterdrückt wird,
- Nachbearbeiten der erstellten Bilder (B1, B2) mittels Filtermodulen (F1, F2), wobei das erste Bild (B1) mit einem ersten Filtermodul (F1) nachbearbeitet wird und das zweite Bild (B2) mit einem zweiten Filtermodul (F2),
- Vereinigen der nachbearbeiteten Bilder (B1, B2) zu einem Gesamtbild (G), bei dem das erste Material und das zweite Material dargestellt sind,
- Ausgeben des Gesamtbildes (G).

Die Erfindung betrifft des Weiteren eine entsprechende sowie eine Steuereinrichtung für ein Radiographie-System und ein Radiographie-System mit einer solchen Steuereinrichtung.

## Beschreibung

Die Erfindung betrifft ein Verfahren und eine Vorrichtung zur Nachbearbeitung von Radiogrammen sowie eine Steuereinrichtung für ein Radiographie-System und ein Radiographie-System mit einer solchen Steuereinrichtung.

Die Radiographie ist ein weit verbreitetes Verfahren in der Medizin, um Aufnahmen des Körperinneren anfertigen zu können. Ein Röntgenstrahl wird durch den Körper hindurch auf einen Detektor (früher einen Film) gestrahlt und es ergibt sich ein Radiogramm bzw. ein Röntgenbild, welches eine Projektion des Körpers auf eine Ebene darstellt. Neben nativen Aufnahmen können auch Aufnahmen nach Verabreichung eines Kontrastmittels, oftmals Jod, erfolgen, z.B. bei einer Angiographie.

Um eine visuelle Interpretation von Radiogrammen zu ermöglichen, werden typischerweise mehrere Bildverarbeitungsschritte durchgeführt. Eine grobe Trennung einer Bildverarbeitungspipeline kann durch die Aufteilung in physikalisch motivierte Korrekturen (z.B. log(I/I0) oder eine Streustrahlkorrektur), eine Dynamikkomprimierung (engl. "Dynamic Range Compression", DRC) und eine (Hochfrequenz-)Detailverstärkung (engl.: High Frequency Feature Enhancement, HFE) erfolgen.

Die Hauptmotivation für den DRC-Schritt besteht darin, die niederfrequente Helligkeitsmodulation eines Bildes zu beseitigen, die durch die variierende projizierte Dicke des Objekts verursacht wird. Die relativen Knochenkontraste innerhalb dieses Objekts müssen dabei erhalten bleiben. Eine Standardmethode hierfür ist die Anwendung eines Tiefpassfilters (Gauß oder bilateral) und die Durchführung einer nichtlinearen Transformation dieses Bildes, die seinen Dynamikbereich reduziert, z. B. durch Anwenden einer S-förmigen Transformationsfunktion. Nach dieser Transformation werden verbleibende hohe Frequenzen wieder hinzugefügt.

Wenn der Tiefpassfilter den Knochen nicht vollständig von der Weichteilstruktur trennen kann, entfernt der DRC automatisch den niederfrequenten Knochenkontrast in Bereichen mit geringerer Objektdicke, was ein gravierendes Problem darstellt. Hier stellen insbesondere Extremitätenbilder eine Herausforderung dar, da der Hintergrund, der ausgeglichen werden muss, extrem variieren kann, z.B. ein Fußbild inklusive der Zehen.

Die Hauptmotivation des HFE-Schritts ist die Verbesserung diagnostisch relevanter Strukturen im Vergleich zu anatomischem Rauschen und Quantenrauschhintergrund.

Bei beiden Filterungsmethoden tritt das Problem auf, dass bei einem Übergang von Regionen mit einem hohen Kontrast zu Regionen mit einem niedrigen Kontrast bei einer automatisierten Filterung ein systematischer Fehler erzeugt werden kann. Bis heute konnte dieses Problem nicht gelöst werden. Die einzige Abhilfe ist die Reduzierung der Stärke von DRC oder HFE, was häufig zu einem typisch "analogen" oder "filmähnlichen" Bildeindruck führt.

Es ist eine Aufgabe der vorliegenden Erfindung, ein alternatives, komfortableres Verfahren und eine entsprechende Vorrichtung zur Nachbearbeitung von Radiogrammen anzugeben, mit dem die oben beschriebenen Nachteile vermieden werden.

Diese Aufgabe wird durch ein Verfahren gemäß Patentanspruch 1, eine Vorrichtung gemäß Patentanspruch 8 sowie durch eine Steuereinrichtung gemäß Patentanspruch 9 und ein Radiographie-System gemäß Patentanspruch 10 gelöst.

Ein erfindungsgemäßes Verfahren dient zur Nachbearbeitung von Radiogrammen aus Radiographiedaten, welche spektrale Aufnahmen eines Motivs eines Objekts, z.B. eines Körperbereichs eines Patienten, umfassen. Ein Röntgen-System, welches solche spektralen Daten aufnehmen kann, ist insbesondere ein Röntgen-System mit photonenzählenden Detektoren, ein Röntgen-System mit einem Dual-Layer Detektor oder Multi-Layer Detektor, ein Röntgensystem das zwei aufeinanderfolgende Bilder mit unterschiedlichen Strahlenergien (kV) und/oder Filtern aufnimmt.

Das Verfahren umfasst die folgenden Schritte:
- Erstellen eines ersten Bildes aus den Radiogrammen, in dem ein erstes Material des Objekts, welches andere spektrale Schwächungseigenschaften besitzt als ein zweites Material des Objekts, hervorgehoben wird und das zweite Material unterdrückt wird,
- Erstellen eines zweiten Bildes aus den Radiogrammen, in dem das zweite Material hervorgehoben wird und das erste Material unterdrückt wird,
- Nachbearbeiten der erstellten Bilder mittels Filtermodulen, wobei das erste Bild mit einem ersten Filtermodul nachbearbeitet wird und das zweite Bild mit einem zweiten Filtermodul,
- Vereinigen der nachbearbeiteten Bilder zu einem Gesamtbild, bei dem das erste Material und das zweite Material dargestellt sind,
- Ausgeben des Gesamtbildes.

Die Erstellung eines Bildes ist dem Fachmann grundsätzlich bekannt. In dem hier geschilderten Fall liegen als Rohdaten Radiogramme vor (z.B. Projektionsbilder) und es findet eine Materialzerlegung basierend auf diesen Radiogrammen statt. Vereinfachend kann hier z.B. die Annahme getroffen werden, dass ein Objekt aus nur zwei Materialien besteht. Die (materialzerlegende) Erstellung liefert dann zwei Bilder.

Oftmals liegen bei spektralen Aufnahmen eines Motivs zwei oder mehr Rohbilder vor, die das Motiv bei jeweils unterschiedlichen Strahlenergien darstellen. Es kann auch ein Datensatz vorliegen, welches an jeder Pixelposition einen Vektor aus zwei oder mehr Bildwerten aufweist. Bei einer Erstellung eines Bildes wird typischerweise der Bildwert jedes Pixels des erstellten Bildes aus einer Verknüpfung basierend auf entsprechenden Pixeln der Rohbilder (bzw. der Vektoreinträge) berechnet. Es kann aber auch für jedes Pixel des resultierenden Bildes eine Pixelgruppe der Rohbilder um das entsprechende Pixel herum betrachtet werden. Bevorzugt umfasst die Berechnung eine gewichtete Subtraktion bei der z.B. aus den Werten A und B von einander entsprechenden Pixeln zweier Rohbilder der Wert X eines entsprechenden Pixels des erstellten Bildes mit den Koeffizienten a und b mit der Formel X = aA - bB berechnet wird. Die Koeffizienten stellen dabei bevorzugt Wichtungen dar. Neben einer Subtraktion kann auch eine nichtlineare Kombination der Eingangswerte berechnet werden. Wahlweise kann anstelle einer analytischen Funktion auch eine Lookup-Tabelle genutzt werden.

Es werden dabei (mindestens) zwei Bilder erstellt, die zwei Materialien des aufgenommenen Objekts unterschiedlich darstellen. Diese beiden Materialien unterscheiden sich dadurch, dass das erste Material andere spektrale Schwächungseigenschaften besitzt (z.B. mehr Strahlung absorbiert) als das zweite Material. Bevorzugt ist dabei das erste Material Knochenmaterial oder ein Kontrastmittel (z.B. Jod) und das zweite Material Gewebe.

In dem ersten Bild wird das erste Material (z.B. Knochen) hervorgehoben und das zweite Material (z.B. umgebendes Gewebe) unterdrückt, in dem zweiten Bild wird genau umgekehrt das zweite Material (z.B. Gewebe) hervorgehoben und das erste Material (z.B. Knochen) unterdrückt. In dem Beispiel Knochen bzw. Gewebe könnte das erste Bild als "Knochenbild" bezeichnet werden und das zweite Bild als VNBC-Bild (VNBC: "Virtual Non-Bone Contrast"). Hierbei sollte beachtet werden, dass im Gegensatz zu "Virtual Non-Calcium" Bildern (VNCa-Bilder) bezüglich der Erfindung bevorzugt eine Zerlegung gezeigt wird, bei der ein lokaler Calciumkontrast durch Wasser ersetzt wird und nicht auf 0 gesetzt wird. Ein Setzen auf "0" (wie im Stand der Technik oft getan) würde im Projektionsbild häufig einen Negativkontrast zeigen, der hier nachteilhaft sein kann.

Da hier von Bildern gesprochen wird, ist es selbstverständlich, dass "Hervorheben" oder "Unterdrücken" eines Materials, dem "Hervorheben" bzw. "Unterdrücken" einer Bildinformation dieses Materials entspricht. Beispielsweise können Werte von Pixeln eines Bildes verringert werden, um ein Material zu unterdrücken oder erhöht werden, um ein Material hervorzuheben. Bei inversen Bildern kann es genau umgekehrt sein.

Die erstellten Bilder (erstes Bild und zweites Bild und ggf. weitere Bilder) werden dann mittels Filtermodulen nachbearbeitet. Diese Filtermodule umfassen bevorzugt die oben genannten DRC-Filter und HFE-Filter, also einen Kompressor und einen Filter zur Detailverstärkung. Wie im einleitenden Teil gesagt, ist die Anwendung dieser Filter auf ein Bild im Stand der Technik bekannt. Der Unterschied zum Stand der Technik liegt hier darin, dass die beiden Bilder durch eine (physikalische) Materialzerlegung entstehen (und nicht durch eine bildbasierte Zerlegung) und das erste Bild mittels eines ersten Filtermoduls nachbearbeitet wird und das zweite Bild mittels eines zweiten Filtermoduls. Diese Filtermodule können damit auf die Art der Bilder optimiert sein, sich also unterscheiden. Beispielsweise kann das erste Filtermodul einen DRC-Filter und einen HFE-Filter umfassen, die auf ein Knochenbild optimiert worden sind und das zweite Filtermodul einen DRC-Filter und einen HFE-Filter, die auf ein VNBC-Bild optimiert worden sind. Zusätzlich können auch weitere Bilder mit weiteren Filtermodulen nachbearbeitet werden, wobei jedes Filtermodul für ein Bild (besser für einen Bild-Typ) verwendet werden soll, für das es optimiert worden ist.

Nachdem jedes Bild nachbearbeitet worden ist, und damit insbesondere eine für sich optimale Filterung erfuhr, werden die nachbearbeiteten Bilder zu einem Gesamtbild vereinigt. Mit den nachbearbeiteten Bildern sind zumindest das erste Bild und das zweite Bild nach ihrer Nachbearbeitung gemeint. Weitere nachbearbeitete Bilder können hinzugenommen werden, sie können aber auch zu einem weiteren Gesamtbild vereinigt werden. Da in dem ersten Bild das erste Material (z.B. Knochen) hervorgehoben ist und im zweiten Bild das zweite Material (z.B. Gewebe), zeigt das Gesamtbild sowohl das erste Material als auch das zweite Material (also z.B. Knochen mit umgebendem Gewebe). Das Gesamtbild wird dann ausgegeben, z.B. zu einer Beurteilung durch eine Person.

Eine erfindungsgemäße Vorrichtung dient zur Nachbearbeitung von Radiogrammen aus Radiographiedaten, welche spektrale Aufnahmen eines Motivs eines Objekts umfassen. Sie ist bevorzugt zur Ausführung des erfindungsgemäßen Verfahrens ausgelegt und umfasst die folgenden Komponenten:
- eine Bildeinheit ausgelegt zum Erstellen eines ersten Bildes aus den Radiogrammen, in dem ein erstes Material des Objekts, welches andere spektrale Schwächungseigenschaften besitzt als ein zweites Material des Objekts, hervorgehoben wird und das zweite Material unterdrückt wird, und zum Erstellen eines zweiten Bildes aus den Radiogrammen, in dem das zweite Material hervorgehoben wird und das erste Material unterdrückt wird,
- eine Nachbearbeitungseinheit ausgelegt zum Nachbearbeiten der erstellten Bilder mittels Filtermodulen, wobei das erste Bild mit einem ersten Filtermodul nachbearbeitet wird und das zweite Bild mit einem zweiten Filtermodul,
- eine Vereinigungseinheit ausgelegt zum Vereinigen der nachbearbeiteten Bilder zu einem Gesamtbild, bei dem das erste Material und das zweite Material dargestellt sind,
- eine Ausgabeeinheit ausgelegt zum Ausgeben des Gesamtbildes.

Die Funktion der Komponenten wurde vorangehend im Rahmen des Verfahrens bereits beschrieben. Die Vorrichtung ist besonders bevorzugt (speziell) zur Durchführung des erfindungsgemäßen Verfahrens ausgelegt.

Eine erfindungsgemäße Steuereinrichtung zur Steuerung eines Radiographie-Systems umfasst eine erfindungsgemäße Vorrichtung und/oder ist zur Ausführung eines erfindungsgemäßen Verfahrens ausgelegt.

Ein erfindungsgemäßes Radiographie-System umfasst eine erfindungsgemäße Steuereinrichtung.

Ein Großteil der zuvor genannten Komponenten der Vorrichtung können ganz oder teilweise in Form von Softwaremodulen in einem Prozessor eines entsprechenden Rechensystems realisiert werden, z.B. von einer Steuereinrichtung eines Radiographie-Systems. Eine weitgehend softwaremäßige Realisierung hat den Vorteil, dass auch schon bisher verwendete Rechensysteme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem ladbar ist, mit Programmabschnitten, um die Schritte des erfindungsgemäßen Verfahrens, zumindest die durch einen Computer ausführbaren Schritte auszuführen, wenn das Programm in dem Rechensystem ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zum Rechensystem bzw. zur Steuereinrichtung und/oder zur Speicherung an oder in dem Rechensystem bzw. der Steuereinrichtung kann ein computerlesbares Medium, z.B. ein Memorystick, eine Festplatte oder ein sonstiger transportabler oder fest eingebauter Datenträger dienen, auf welchem die von einem Rechensystem einlesbaren und ausführbaren Programmabschnitte des Computerprogramms gespeichert sind. Das Rechensystem kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Gemäß einem bevorzugten Verfahren ist das erste Material Knochen und/oder ein Kontrastmittel, insbesondere Jod. Das zweite Material ist bevorzugt (umliegendes) Gewebe. Das erste Bild wird bevorzugt dermaßen konstruiert, dass Knochen eines Patienten und/oder das Kontrastmittel hervorgehoben werden und die Information des umliegenden Gewebes des Patienten unterdrückt wird. Das zweite Bild wird bevorzugt dermaßen konstruiert, dass das Gewebe des Patienten hervorgehoben wird und die Information von Knochen des Patienten und/oder das Kontrastmittel unterdrückt wird.

Wie bereits gesagt, werden bei einer Hervorhebung eines Materials Bildwerte von Pixeln, die dieses Material zeigen, erhöht (bzw. in einem invertierten Bild verringert). Bei einer Unterdrückung eines Materials werden Bildwerte von Pixeln, die dieses Material zeigen, verringert (bzw. in einem invertierten Bild erhöht).

Gemäß einem bevorzugten Verfahren liegen bei einer spektralen Aufnahme eines Motivs (z.B. eines Körperbereichs eines Patienten) eine Mehrzahl von Rohbildern als Radiogramme vor, bzw. werden diese erstellt. Das erste Bild und das zweite Bild werden dann bevorzugt durch eine Anzahl gewichteter Subtraktion zweier Rohbilder berechnet, wobei jeweils ein Gewichtungsfaktor definiert, welches Material in diesen Bildern hervorgehoben und welches Material unterdrückt wird. Dies kann z.B. durch die oben genannte Funktion X = aA - bB für die einzelnen Bildpunkte geschehen, wobei a und b die Gewichtungsfaktoren sind. Die beiden Bilder werden dabei aus denselben Rohbildern erstellt.

Gemäß einem bevorzugten Verfahren zeigt das zweite Bild eine Zerlegung von Rohbildern, bei der ein lokaler Kontrast, insbesondere ein Calciumkontrast und/oder ein Kontrastmittel-Kontrast, insbesondere ein Jodkontrast, virtuell durch Wasser ersetzt wurde. Dies hat, wie oben gesagt den Vorteil, dass ein Negativkontrast verhindert wird.

Gemäß einem bevorzugten Verfahren resultieren die mindestens zwei unterschiedlichen Strahlenergien einer spektralen Aufnahme aus Beschleunigungsspannungen, die einen Unterschied von mindestens 30 kV haben, insbesondere mindestens 50 kV. Alternativ oder ergänzend (z.B. wenn mit einem breiten Röntgenspektrum bestrahlt wird) haben die mittlere Strahlenergien bevorzugt einen Unterschied von mindestens 30 keV, insbesondere mindestens 50 keV.

Gemäß einem bevorzugten Verfahren umfasst ein Filtermodul einen Kompressor (einen DRC-Filter), welcher den Dynamikumfang des Bildes komprimiert. Ein Kompressor beseitigt die niederfrequente Helligkeitsmodulation eines Bildes, die durch die variierende projizierte Dicke des Objekts verursacht wird. Eine Standardmethode hierfür ist die Anwendung eines Tiefpassfilters (Gauß oder bilateral) und die Durchführung einer nichtlinearen Transformation dieses Bildes, die seinen Dynamikbereich reduziert, z. B. durch Anwenden einer S-förmigen Transformationsfunktion. Nach dieser Transformation werden verbleibende hohe Frequenzen wieder hinzugefügt.

Gemäß einem bevorzugten Verfahren umfasst ein Filtermodul einen Detailverstärkungs-Filter ("HFE-Filter" für "High Frequency Feature Enhancement"). Ein HFE-Filter verbessert diagnostisch relevante Strukturen im Vergleich zu anatomischem Rauschen und Quantenrauschhintergrund. Bevorzugt ist auch eine Serienanwendung eines Kompressors (DRC-Filter) und eines Detailverstärkungs-Filters (HFE-Filter).

Gemäß einem bevorzugten Verfahren umfasst ein Filtermodul einen Entrauschungsfilter. Solche Filter sind im Stand der Technik bekannt.

Gemäß einem bevorzugten Verfahren unterscheidet sich das erste Filtermodul vom zweiten Filtermodul. Die Filtermodule sollten für die Bilder, auf die sie angewendet werden, optimiert sein. So sollte das erste Filtermodul für das erste Bild für erste Strukturen optimiert sein, also für Strukturen, die relativ stark absorbieren. Das zweite Filtermodul für das zweite Bild sollte für zweite Strukturen optimiert sein, also für Strukturen, die relativ schwach absorbieren. Es ist dabei bevorzugt, dass die Filtermodule die gleichen Typen von Filtern und/oder die gleiche Architektur von Filtern aufweisen, jedoch Parameter der Filter unterschiedlich sind. Ein bevorzugtes Beispiel ist, dass beide Filter einen DRC-Filter und einen HFE-Filter aufweisen (und ggf. noch einen Entrauschungsfilter), die insbesondere auch in derselben Reihenfolge angewandt werden.

Bevorzugt ist der Einsatz von KI-basierten Verfahren (KI: "Künstliche Intelligenz") für das erfindungsgemäße Verfahren. Eine künstliche Intelligenz basiert auf dem Prinzip des maschinenbasierten Lernens, und wird in der Regel mit einem lernfähigen Algorithmus durchgeführt der entsprechend trainiert worden ist. Für maschinenbasiertes Lernen wird häufig der englische Ausdruck "Machine Learning" verwendet, wobei hier auch das Prinzip des "Deep Learning" mit umfasst wird. Beispielsweise wird ein Deep Convolutional Neural Network (DCNN) darauf trainiert, aus zwei Radiogrammen als Rohbilder das erste und das zweite Bild zu erstellen. Aber auch die Filter eines Filtermoduls oder auch ein Filtermodul können durch eine KI realisiert werden.

Bevorzugt liegen Komponenten der Erfindung, insbesondere die Bildeinheit und/oder die Nachbearbeitungseinheit als ein "Cloud-Dienst" vor. Ein solcher Cloud-Dienst dient der Bearbeitung von Daten, insbesondere mittels einer künstlichen Intelligenz, kann aber auch ein Dienst basierend auf herkömmlichen Algorithmen sein oder ein Dienst, bei dem im Hintergrund eine Auswertung durch Menschen stattfindet. Generell ist ein Cloud-Dienst (im Folgenden auch kurz als "Cloud" bezeichnet) eine IT-Infrastruktur, bei der über ein Netzwerk z.B. Speicherplatz oder Rechenleistung und/oder eine Anwendungssoftware zur Verfügung gestellt wird. Die Kommunikation zwischen dem Anwender und der Cloud erfolgt dabei mittels Datenschnittstellen und/oder Datenübertragungs-protokollen. Im hier vorliegenden Fall ist besonders bevorzugt, dass der Cloud-Dienst sowohl Rechenleistung als auch Anwendungssoftware zur Verfügung stellt.

Im Rahmen eines bevorzugten Verfahrens erfolgt eine Bereitstellung von Daten über das Netzwerk an den Cloud-Dienst. Dieser umfasst ein Rechensystem, z.B. einen Computercluster, das in der Regel nicht den lokalen Rechner des Benutzers umfasst. Diese Cloud kann insbesondere durch die medizinische Einrichtung, die auch die medizintechnischen Systeme bereitstellt, zur Verfügung gestellt werden. Beispielsweise werden die Daten einer Bildaufnahme über ein RIS (Radiologieinformationssystem) oder PACS an ein (Remote-) Rechnersystem (die Cloud) gesendet. Bevorzugt stellen das Rechensystem der Cloud, das Netzwerk sowie das Radiographie-System einen Verbund im datentechnischen Sinne dar. Das Verfahren kann dabei mittels einer Befehlskonstellation in dem Netzwerk realisiert werden. Die in der Cloud berechneten Daten ("Ergebnisdaten") werden später wieder über das Netzwerk zu dem lokalen Rechner des Anwenders gesendet.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 ein Beispiel für ein Radiographie-System mit einer erfindungsgemäßen Steuereinrichtung,
Figur 2 eine Skizze einer Bildnachbearbeitung gemäß dem Stand der Technik,
Figur 3 eine Skizze einer Bildnachbearbeitung gemäß dem erfindungsgemäßen Verfahren.

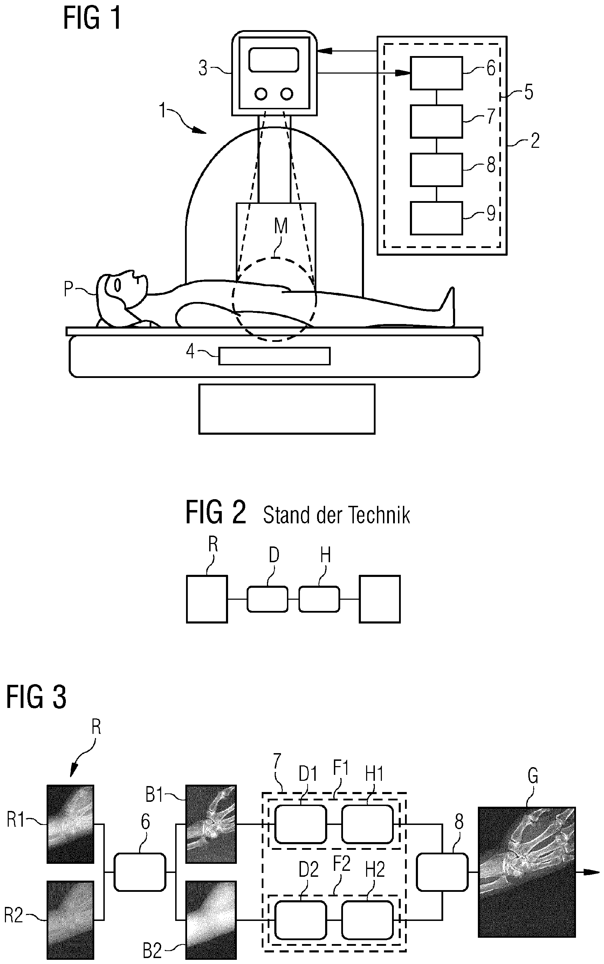

Figur 1 zeigt grob schematisch ein Radiographie-System 1 mit einer Steuereinrichtung 2. Diese ist mit einer Vorrichtung 5 ausgelegt zur Durchführung des erfindungsgemäßen Verfahrens ausgestattet. Das Radiographie-System 1 weist in üblicher Weise eine Strahlungsquelle 3 auf, welche hier eine Röntgenquelle darstellt, und während einer Radiographieaufnahme einen Patienten P durchstrahlt, so dass die Strahlung auf einen der Strahlungsquelle 3 jeweils gegenüberliegenden Detektor 4 trifft. Das Radiographie-System 1 ist dabei dazu ausgelegt, spektrale Aufnahmen anzufertigen und ist z.B. ein System, welches zwei unterschiedliche Beschleunigungsspannungen für die Strahlung erzeugen kann oder einen Detektor 4 hat, der energieaufgelöst messen kann. Das Radiographie-System 1 kann auch selektive Filter umfassen, welche zur Filterung von zwei unterschiedlichen Strahlenergien verwendet werden können.

Bei der Steuereinrichtung 2 sind nur die Komponenten dargestellt, die für die Erläuterung der Erfindung wesentlich sind. Grundsätzlich sind Radiographie-Systeme 1 und zugehörige Steuereinrichtungen 2 dem Fachmann bekannt und brauchen daher nicht im Detail erläutert zu werden.

Die Steuereinrichtung 2 umfasst eine Vorrichtung 5 zur Nachbearbeitung von Radiogrammen R aus Radiographiedaten, welche spektrale Radiogramme eines Motivs M eines Objekts P, z.B. wie hier dargestellt einem Körperbereich M eines Patienten P, bei zwei unterschiedlichen Strahlenergien umfassen.

Die Vorrichtung umfasst eine Bildeinheit 6, eine Nachbearbeitungseinheit 7, eine Vereinigungseinheit 8 und eine Ausgabeeinheit 9, die durch eine einfache Datenschnittstelle realisiert sein kann. Zur Beschreibung der Funktion der Komponenten siehe auch Figur 3.

Die Bildeinheit 6 ist zum Erstellen von mindestens zwei unterschiedlichen Bildern B1, B2 aus den Radiogrammen R ausgelegt. Das erste Bild B1 zeigt dabei die Knochen des Patienten P prominent und das Gewebe ist unterdrückt. Das zweite Bild B2 zeigt umgekehrt das Gewebe und weniger die Knochen. Anstelle der Knochen kann aber auch ein Kontrastmittel berücksichtigt werden, z.B. Jod.

Die Nachbearbeitungseinheit 7 ist zum Nachbearbeiten der erstellten Bilder B1, B2 mittels Filtermodulen F1, F2 ausgelegt, wobei das erste Bild B1 mit einem ersten Filtermodul F1 nachbearbeitet wird und das zweite Bild B2 mit einem zweiten Filtermodul F2. Diese Filtermodule F1, F2 können sich dabei durchaus unterscheiden, was den besonderen Vorteil der Erfindung ausmacht. Jedes der Bilder B1, B2 kann nämlich mit auf diese Bilder B1, B2 optimierten Filtermodulen F1, F2 nachbearbeitet werden. Es ist dabei bevorzugt, dass die Filtermodule F1, F2 die gleichen Typen von Filtern D1, D2, H1, H2 und/oder die gleiche Architektur von Filtern D1, D2, H1, H2 aufweisen, jedoch Parameter der Filter D1, D2, H1, H2 unterschiedlich sind.

Die Vereinigungseinheit 8 ist zum Vereinigen der nachbearbeiteten Bilder B1, B2 zu einem Gesamtbild G ausgelegt. Bei diesem Gesamtbild sind das erste Material und das zweite Material dargestellt.

Die Ausgabeeinheit 9 dient zum Ausgeben des Gesamtbildes G, z.B. zur Befundung.

Figur 2 zeigt eine Skizze einer Bildnachbearbeitung gemäß dem Stand der Technik. In dem dargestellten Beispiel wird ein Radiogramm R (alle aufgenommenen Bilder) mit denselben Filtern D, H nachbearbeitet.

Figur 3 skizziert ein Beispiel für das erfindungsgemäße Verfahren zur Nachbearbeitung von spektralen Radiogrammen R aus Radiographiedaten, die z.B. mit einem Radiographie-System 1 nach Figur 1 bei zwei unterschiedlichen Strahlenergien aufgenommen worden sind. Es ergaben sich als Radiogramm R zwei Rohbilder R1, R2, die ganz links dargestellt sind. Die Strahlenergien sollten recht weit auseinanderliegen und sich nicht überlappen, damit unterschiedliche Informationen in den Rohbildern R1, R2 vorliegen.

Mittels der Bildeinheit 6 werden nun ein erstes Bild B1 und ein zweites Bild B2 aus den Rohbildern R1, R2 erstellt und dabei bestimmte Materialien unterdrückt bzw. hervorgehoben. In diesem Beispiel ist das erste Material Knochen und das zweite Material umliegendes Gewebe. Das erste Bild B1 wird dabei dermaßen konstruiert, dass die Knochen hervorgehoben werden und die Information des umliegenden Gewebes des Patienten P unterdrückt wird, und das zweite Bild B2 wird dermaßen konstruiert, dass das Gewebe hervorgehoben wird und die Information von Knochen unterdrückt wird.

Dann erfolgt eine Nachbearbeitung der erstellten Bilder B1, B2 mittels Filtermodulen F1, F2 der Nachbearbeitungseinheit 7. In diesem Beispiel umfasst jedes Filtermodul F1, F2 einen Kompressor D1, D2 (bzw. einen DRC-Filter D1, D2), welcher den Dynamikumfang des Bildes komprimiert und einen Detailverstärkungs-Filter H1, H2 (bzw. einen HFE-Filter H1, H2). Dabei wird das erste Bild B1 mit dem ersten Filtermodul F1 nachbearbeitet und das zweite Bild B2 mit dem zweiten Filtermodul F2.

Nach dieser Nachbearbeitung werden die nachbearbeiteten Bilder B1, B2 mit der Vereinigungseinheit 8 zu einem Gesamtbild G vereinigt und ausgegeben.

Es wird abschließend noch einmal darauf hingewiesen, dass es sich bei den vorhergehend detailliert beschriebenen Figuren lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Gerät" nicht aus, dass die betreffenden Komponenten aus mehreren zusammenwirkenden Teil-Komponenten bestehen können, die gegebenenfalls auch räumlich verteilt sein können. Der Ausdruck "eine Anzahl" ist als "mindestens eins" zu verstehen. Unabhängig vom grammatikalischen Geschlecht eines bestimmten Begriffes sind Personen mit männlicher, weiblicher oder anderer Geschlechteridentität mit umfasst.

## Patentansprüche

1. Verfahren zur Nachbearbeitung von Radiogrammen (R) aus Radiographiedaten, welche spektrale Aufnahmen eines Motivs (M) eines Objekts (P) umfassen, das Verfahren umfassend die Schritte:
- Erstellen eines ersten Bildes (B1) aus den Radiogrammen (R), in dem ein erstes Material des Objekts (P), welches andere spektrale Schwächungseigenschaften besitzt als ein zweites Material des Objekts (P), hervorgehoben wird und das zweite Material unterdrückt wird,
- Erstellen eines zweiten Bildes (B2) aus den Radiogrammen (R), in dem das zweite Material hervorgehoben wird und das erste Material unterdrückt wird,
- Nachbearbeiten der erstellten Bilder (B1, B2) mittels Filtermodulen (F1, F2), wobei das erste Bild (B1) mit einem ersten Filtermodul (F1) nachbearbeitet wird und das zweite Bild (B2) mit einem zweiten Filtermodul (F2),
- Vereinigen der nachbearbeiteten Bilder (B1, B2) zu einem Gesamtbild (G), bei dem das erste Material und das zweite Material dargestellt sind,
- Ausgeben des Gesamtbildes (G).

2. Verfahren nach Anspruch 1, wobei das erste Material Knochen und/oder ein Kontrastmittel ist und das zweite Material umliegendes Gewebe, wobei das erste Bild (B1) dermaßen konstruiert wird, dass Knochen eines Patienten (P) und/oder das Kontrastmittel hervorgehoben werden und die Information des umliegenden Gewebes des Patienten (P) unterdrückt wird, und wobei das zweite Bild (B2) dermaßen konstruiert wird, dass das Gewebe des Patienten (P) hervorgehoben wird und die Information von Knochen des Patienten (P) und/oder dem Kontrastmittel unterdrückt wird.

3. Verfahren nach einem der vorangehenden Ansprüche, wobei bei einer spektralen Aufnahmen eines Motivs (M) eine Mehrzahl von Rohbildern (R1, R2) als Radiogramme (R) vorliegen oder erstellt werden und das erste Bild (B1) und das zweite Bild (B2) durch eine Anzahl gewichteter Subtraktion zweier Rohbilder (R1, R2) berechnet werden, wobei jeweils ein Gewichtungsfaktor definiert, welches Material in diesen Bildern (B1, B2) hervorgehoben und welches Material unterdrückt wird.

4. Verfahren nach einem der vorangehenden Ansprüche, wobei das zweite Bild (B2) eine Zerlegung von Rohbildern (R1, R2) zeigt, bei der ein lokaler Kontrast, insbesondere ein Calciumkontrast und/oder Kontrastmittel-Kontrast, virtuell durch Wasser ersetzt wurde.

5. Verfahren nach einem der vorangehenden Ansprüche, wobei die Radiogramme (R) mit einem Röntgen-System
- mit photonenzählenden Detektoren, und/oder
- mit einem Dual-Layer Detektor, und/oder
- mit einem Multi-Layer Detektor, und/oder
- welches zwei aufeinanderfolgende Bilder mit unterschiedlichen Strahlenergien aufnimmt, und/oder
- welches zwei aufeinanderfolgende Bilder mit unterschiedlichen Filtern aufnimmt
aufgenommen werden.

6. Verfahren nach einem der vorangehenden Ansprüche, wobei ein Filtermodul (F1, F2) einen Kompressor (D1, D2) umfasst, welcher den Dynamikumfang des Bildes komprimiert und/oder einen Detailverstärkungs-Filter (H1, H2) umfasst und/oder einen Entrauschungsfilter umfasst, bevorzugt eine Serienanwendung von einem Kompressor (D1, D2) und einem Detailverstärkungs-Filter (H1, H2).

7. Verfahren nach einem der vorangehenden Ansprüche, wobei sich das erste Filtermodul (F1) vom zweiten Filtermodul (F1) unterscheidet, bevorzugt wobei die Filtermodule (F1) die gleichen Typen von Filtern (D1, D2, H1, H2) und/oder die gleiche Architektur von Filtern (D1, D2, H1, H2) aufweisen, jedoch Parameter der Filter (D1, D2, H1, H2) unterschiedlich sind.

8. Vorrichtung (5) zur Nachbearbeitung von Radiogrammen (R) aus Radiographiedaten, welche spektrale Aufnahmen eines Motivs (M) eines Objekts (P) umfassen, die Vorrichtung umfassend:
- eine Bildeinheit (6) ausgelegt zum Erstellen eines ersten Bildes (B1) aus den Radiogrammen (R), in dem ein erstes Material des Objekts (P), welches andere spektrale Schwächungseigenschaften besitzt als ein zweites Material des Objekts (P), hervorgehoben wird und das zweite Material unterdrückt wird, und zum Erstellen eines zweiten Bildes (B2) aus den Radiogrammen (R), in dem das zweite Material hervorgehoben wird und das erste Material unterdrückt wird,
- eine Nachbearbeitungseinheit (7) ausgelegt zum Nachbearbeiten der erstellten Bilder (B1, B2) mittels Filtermodulen (F1, F2), wobei das erste Bild (B1) mit einem ersten Filtermodul (F1) nachbearbeitet wird und das zweite Bild (B2) mit einem zweiten Filtermodul (F2),
- eine Vereinigungseinheit (8) ausgelegt zum Vereinigen der nachbearbeiteten Bilder (B1, B2) zu einem Gesamtbild (G), bei dem das erste Material und das zweite Material dargestellt sind,
- eine Ausgabeeinheit (9) ausgelegt zum Ausgeben des Gesamtbildes (G).

9. Steuereinrichtung (2) zur Steuerung eines Radiographie-Systems (1) umfassend eine Vorrichtung (5) nach Anspruch 8 und/oder ausgelegt zur Ausführung eines Verfahrens nach einem der Ansprüche 1 bis 7.

10. Radiographie-System (1) umfassend eine Steuereinrichtung (2) nach Anspruch 9.

11. Computerprogrammprodukt, umfassend Befehle, die bei der Ausführung des Programms durch einen Computer diesen veranlassen, die Schritte des Verfahrens nach Anspruch 1 bis 7 auszuführen.

12. Computerlesbares Speichermedium, umfassend Befehle, die bei der Ausführung durch einen Computer diesen veranlassen, die Schritte des Verfahrens nach Anspruch 1 bis 7 auszuführen.
